# EUROPEAN PATENT APPLICATION

(11) **EP 3 995 144 A1**
(43) Date of publication of application: **11.05.2022**
(21) Application number: 20874538.0
(22) Date of filing: 18.09.2020
(51) Int. Cl.: A61K 38/20, A61K 45/06, A61P 35/00, G01N 33/68

(54) **COMPOSITION COMPRISING INHIBITOR AGAINST EXPRESSION OF EXOSOMAL PD-L1 AS ACTIVE INGREDIENT FOR ENHANCING ANTICANCER EFFECT**

(30) Priority: 11.10.2019 KR 20190126174; 15.09.2020 KR 20200118558
(71) Applicant: Kyungpook National University Industry-Academic Cooperation Foundation, Daegu 41566 (KR); Daegu Gyeongbuk Institute Of Science and Technology, Daegu 42988 (KR)
(72) Inventor: BAEK, Moon-Chang, Daegu 42116 (KR); YEA, Kyungmoo, Dalseong-gun Daegu 43016 (KR); JUNG, Do Kyung, Dalseong-gun Daegu 42995 (KR); RYU, Suyeon, Daegu 42605 (KR); NOH, Soo Jeong, Daejeon 35212 (KR)
(74) Representative: Roos, Peter
(86) International application number: PCT/KR2020/012631
(87) International publication number: WO 2021/071127

(57) **Abstract**

The present invention relates to a composition containing IL-2 or an expression promoter or activator thereof as an active ingredient for prevention or treatment of cancer disease. In the present invention, it was discovered that when treating cancer cells with IL-2, an anticancer effect was enhanced by inhibiting the secretion of cancer cell-derived exosomes and the expression of PD-L1 inducing immune escape of T cells. IL-2 was also discovered to effectuate the anticancer effect directly on cancer cells compared to conventional techniques exhibiting an immune anticancer effect by activating cytotoxic T cells. Thus, IL-2 or an expression promoter or activator thereof can be advantageously used in a composition for prevention or treatment of cancer diseases.

<110>    Kyungpook National University
         Daegu Gyeongbuk Institute of Science and Technology

<120>    Composition for enhancing anticancer effect comprising an
         inhibitor for the expression of exosomal PD-L1 as an active
         ingredient

<130>    AOP-2020-0049PCT

<160>    4

<170>    KoPatentIn 3.0

<210>    1
<211>    20
<212>    DNA
<213>    Artificial Sequence

<220>
<223>    PD-1-f


<400>    1
agttcccaaa acatgaggat                                                   20


<210>    2
<211>    20
<212>    DNA
<213>    Artificial Sequence

<220>
<223>    PD-1-r


<400>    2
cacgtacaag tcctttggag                                                   20


<210>    3
<211>    20
<212>    DNA
<213>    Artificial Sequence

<220>
<223>    PD-L1-f


<400>    3
ggtcatccca gaactacctc                                                   20


<210>    4
<211>    20
<212>    DNA
<213>    Artificial Sequence

<220>
<223>    PD-L1-r


<400>    4
acggaagatg aatgtcagtg                                                   20

## Description

### TECHNICAL FIELD

The present invention relates to a composition for enhancing an anticancer effect containing an inhibitor against expression of exosomal PD-L1 as an active ingredient, and more specifically, relates to an anticancer effect enhancer using a novel mechanism by which IL-2 inhibits the expression of exosomal PD-L1 in cancer cells.

### BACKGROUND ART

Melanoma is an aggressive skin cancer with an ever-increasing incidence worldwide. This disease primarily affects the white population of both sexes with high rates of metastasis, mortality, and recurrence. Several therapies have been approved by the US Food and Drug Administration (FDA) and European Medicines Agency (EMA) over the past few years. Depending on the characteristics of the tumor, treatment may be surgical resection, chemotherapy, radiation therapy, photodynamic therapy (PDT), immunotherapy, or targeted therapy. Approval of BRAF protein inhibitors such as vemurafenib and dabrafenib, or targeted therapies with MEK inhibitors typified by trametinib, and immune checkpoint inhibitors such as nivolumab, ipilimumab, and pembrolizumab has been introduced. In addition, cytokine IL-2 has been used extensively as a single agent for host immune modulation in cancer therapy.

IL-2 is the most successful treatment of melanoma as a biological response modifier. It is an important factor that promotes T cell activation, proliferation, and cytotoxic activity of CD8+ T cells and NK cells and regulates T cell differentiation programs in response to antigens. The mechanism of immune cells is well known for IL-2, the most studied cytokine since its discovery about 38 years ago, but the mechanisms of melanoma and IL-2 are unclear.

Small extracellular vesicles (sEVs), which are membrane-bound vesicles with a diameter of 40 to 150 nm, include exosomes and microvesicles, and are present in almost all biological fluids. sEVs contain functional biomolecules (i.e., proteins, lipids, RNA, and DNA) that can migrate horizontally to recipient cells. After fusing with plasma membrane, they are released into the extracellular space in most cell types.

Tumor cells can evade the immune system by many strategies. Among the strategies, they induce immune escape by inducing surface expression of programmed death-ligand 1 (PD-L1), which communicates with programmed death 1 (PD-1). Exosomal PD-L1 can interact with PD-1 and help cancer survival by inhibiting activation of T cells. It is clinically important because it affects the progression of cancer. Tumor-derived extracellular vesicles play an important role in increasing aggression and metastatic in intracellular signaling and epithelial-mesenchymal transition (EMT). Many factors regulating extracellular vesicle secretion are already known. Among them, IL-2 and IL-12 may play important roles through regulation of formation and secretion of extracellular vesicles in T cells. However, studies on secretion regulators of tumor-derived exosomal PD-L1 are not clear.

Meanwhile, in addition to surgery, melanoma treatment includes immunotherapy (interferon), chemotherapy, radiation therapy, targeted therapy currently in the introduction stage, and immunotherapy, which is in the spotlight. Immunotherapy is used to treat metastatic melanoma based on the fact that an immune response is involved in the development of melanoma. To date, treatments approved by the US Food and Drug Administration (FDA) include interferon-alpha (for stage 3 treatment) and interleukin-2 (for stage 4 treatment).

As anticancer drugs, dacarbazine (DTIC) and cisplatin are used alone or in combination therapy. Recently, new therapeutic agents known as 'targeted therapy' or 'cell therapy' are being developed, giving hope. Although they are being used gradually by obtaining FDA approval one after another, they are currently in the stage of clinical trials or just before introduction in Korea.

The present invention suggests that more effective anticancer treatment is possible by inhibiting extracellular vesicle secretion and PD-L1 expression in a MAPK signaling-dependent manner in melanoma cells using IL-2, which is used as a treatment for stage 4 melanoma.

### DISCLOSURE

### TECHNICAL PROBLEM

The present invention relates to a composition for enhancing an anticancer effect containing an inhibitor against expression of exosomal PD-L1 as an active ingredient, and more specifically, relates to an anticancer effect enhancer using a novel mechanism by which IL-2 inhibits the expression of exosomal PD-L1 in cancer cells. In the case that cancer cells were treated with IL-2, it was discovered that the anticancer effect of suppressing the secretion of exosomes derived from the cancer cells and suppressing the expression of PD-L1 inducing immune evasion of T cells was enhanced. Accordingly, an object of the present invention is to provide IL-2, or an expression promoter or activator thereof as an inhibitor against the expression of exosomal PD-L1 and as an anticancer effect enhancer capable of enhancing prevention or treatment of cancer diseases.

### TECHNICAL SOLUTION

The present invention provides a composition for enhancing an anticancer effect, containing an inhibitor against expression of exosomal PD-L1 as an active ingredient.

Further, the present invention provides a pharmaceutical composition, containing the composition for enhancing the anticancer effect as an active ingredient.

Furthermore, the present invention provides a method for screening an inhibitor against secretion of cancer cell-derived exosomes and expression of PD-L1 including treating samples isolated from an individual suspected of having a cancer disease with candidate drugs, measuring expression or activity level of IL-2 in the samples treated with the candidate drugs, and selecting one of the candidate drugs that increases the expression or activity level of IL-2 as compared to a control sample.

In addition, the present invention provides a method for inhibiting secretion of cancer cell-derived exosomes and expression of PD-L1, including administering IL-2, or an expression promoter or activator thereof to an individual.

### ADVANTAGEOUS EFFECTS

According to embodiments of the present invention, it was determined that, in the case that cancer cells were treated with IL-2, the anticancer effect of suppressing the secretion of cancer cell-derived exosomes and suppressing the expression of PD-L1 inducing immune evasion of T cells was enhanced. Therefore, the present invention provides IL-2, or an expression promoter or activator thereof as an inhibitor against the expression of exosomal PD-L1 and as an anticancer effect enhancer capable of enhancing prevention or treatment of cancer diseases.

### BRIEF DESCRIPTIONS OF THE DRAWINGS

FIG. 1 shows expression of PD-L1 in the case of directly treating melanoma cells with IL-2.
FIG. 2 shows expression of PD-L1 in the case of directly treating various melanoma cells with IL-2.
FIG. 3 shows images of exosomes obtained by transmission electron microscopy.
FIG. 4 shows results of confirming decreases in expression of exosomal PD-L1 in melanoma cells by IL-2.
FIG. 5 shows results of confirming decreases in the amount of exosomes secreted from melanoma cells and expression of Rab27a by IL-2.

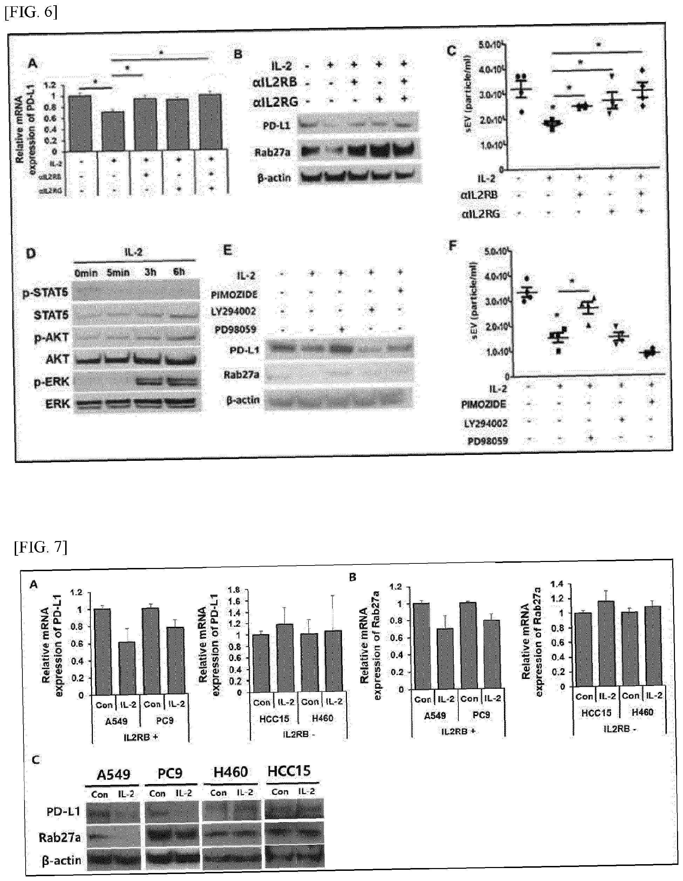

FIG. 6 shows results of confirming a mechanism of PD-L1 expression reduced by IL-2.
FIG. 7 shows changes in expression of PD-L1 and Rab27a in various cancer cells according to IL-2 receptors.
FIG. 8 shows apoptosis of melanoma in co-culture of melanoma cells treated with IL-2 and cytotoxic T cells.
FIG. 9 shows apoptosis of melanoma in co-culture in the case that a subsignal of melanoma cells treated with IL-2 is inhibited in cytotoxic T cells.
FIG. 10 shows results of confirming cancer growth in a melanoma transplanted mouse model.
FIG. 11 shows results of confirming a decrease in PD-L1 expression by IL-2 in a melanoma transplanted mouse model.

### BEST MODE

The terms used herein have been selected as currently widely used general terms as possible while considering the functions in the present invention, but may vary depending on the intention of a person skilled in the art or precedent, the emergence of new technology, or the like. Further, in a specific case, there is a term arbitrarily selected by the applicant, and in this case, its meaning will be described in detail in the description of the corresponding invention. Therefore, the term used herein should be defined based on the meaning of the term and the overall content of the present invention, rather than simply the name of the term.

Unless defined otherwise, all terms used herein, including technical or scientific terms, have the same meaning as commonly understood by persons skilled in the art to which this invention pertains. Terms commonly used such as those defined in dictionaries should be construed as having meanings consistent with the meanings in the context of the related art, and are not to be construed in ideal or overly formal meanings unless explicitly defined in the present application.

A numerical range is inclusive of numerical values defined in the range. Every maximum numerical limitation given throughout this specification includes any lower numerical limitation as if the lower numerical limitation were expressly written. Every minimum numerical limitation given throughout this specification includes any higher numerical limitation as if the higher numerical limitation were expressly written. Every numerical limitation given throughout this specification will include any narrower numerical range within a broader numerical range, as if the narrower numerical limitation were expressly written.

Hereinafter, the present invention will be described in more detail.

The inventors of the present invention determined that, in the case of treating melanoma cells with IL-2, the secretion of cancer-cell derived exosomes was suppressed and the expression of PD-L1 inducing immune evasion of T cells was suppressed, and has completed the present invention by discovering a new mechanism by which IL-2 directly affects on cancer cells to enhance an anticancer effect.

The present invention provides a composition for enhancing the anticancer effect, containing an inhibitor against the expression of exosomal PD-L1 as an active ingredient.

The inhibitor may be IL-2, or an expression promoter or activator thereof, and the IL-2 expression promoter or activator may be selected from the group consisting of compounds, peptides, aptamers, and antibodies that specifically bind to IL-2 proteins. However, the present invention is not limited thereto.

The IL-2, or the expression promoter or activator thereof can suppress the immune evasion and enhance the anticancer effect by reducing the secretion of cancer-cell derived exosomes and the expression of PD-L1.

The cancer may be selected from the group consisting of melanoma, skin cancer, lung cancer, liver cancer, stomach cancer, pancreatic cancer, bone cancer, head or cervical cancer, uterine cancer, ovarian cancer, breast cancer, fallopian tube carcinoma, endometrial carcinoma, cervical carcinoma, vaginal carcinoma, vulvar carcinoma, Hawkins' disease, esophageal cancer, small intestine cancer, colorectal cancer, colon cancer, rectal cancer, perianal cancer, endocrine gland cancer, thyroid cancer, parathyroid cancer, adrenal cancer, soft tissue sarcoma, urethral cancer, penile cancer, prostate cancer, chronic or acute leukemia, lymphocyte lymphoma, bladder cancer, kidney or ureteral cancer, renal cell carcinoma, renal pelvic carcinoma, central nervous system tumor, primary central nervous system lymphoma, spinal cord tumor, brainstem glioma, and pituitary adenoma, but is not limited thereto.

The composition for enhancing the anticancer effect may be administered in combination with one or more anticancer agents.

Further, the present invention provides a pharmaceutical composition containing the composition for enhancing the anticancer effect as an active ingredient.

For administration, the pharmaceutical composition of the present invention may include a pharmaceutically acceptable carrier, excipient, or diluent, in addition to the active ingredient described above. The carrier, excipient, and diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

The pharmaceutical composition of the present invention may be formulated and used in an oral dosage form such as powders, granules, tablets, capsules, suspensions, emulsions, syrups, or aerosols, or in the form of external preparations, suppositories, or sterile injection solutions, according to conventional methods, respectively. Specifically, when formulating, the diluent or excipient may be used such as a filler, a bulking agent, a binder, a wetting agent, a disintegrant, and a surfactant commonly used. Solid preparations for oral administration include, but are not limited to, tablets, pills, powders, granules, and capsules. Such a solid preparation may be prepared by mixing at least one or more excipients, for example, starch, calcium carbonate, sucrose, lactose, and gelatin, in addition to the active ingredient. In addition to simple excipients, lubricants such as magnesium stearate and talc may also be used. It may be prepared by adding various excipients, such as wetting agents, sweetening agents, fragrances, and preservatives, in addition to liquids for oral use and liquid paraffin. Formulations for parenteral administration include sterile aqueous solutions, non-aqueous solvents, suspensions, emulsions, lyophilized formulations, and suppositories. As the non-aqueous solvent and suspension, propylene glycol, polyethylene glycol, vegetable oil such as olive oil, and injectable esters such as ethyl oleate may be used. As the base of the suppository, Witepsol, Macrosol, Tween 61, cacao butter, laurin fat, and glycerogelatin may be used.

A suitable dosage of the pharmaceutical composition of the present invention varies depending on the patient's condition and weight, the degree of disease, drug form, and time, but may be appropriately selected by those skilled in the art. The daily dosage of the composition is preferably 0.001 mg/kg to 50 mg/kg, and may be administered once a day or divided into several times a day as needed.

Further, the present invention provides a method for screening an inhibitor against secretion of cancer cell-derived exosomes and expression of PD-L1 including treating samples isolated from an individual suspected of having a cancer disease with candidate drugs, measuring expression or activity level of IL-2 in the samples treated with the candidate drugs, and selecting one of the candidate drugs that increases the expression or activity level of IL-2 as compared to a control sample.

The expression or activity level of IL-2 may be measured by one or more selected from the group consisting of reverse transcription-polymerase chain reaction (RT-PCR), enzyme-linked immunosorbent assay (ELISA), radioimmunoassay, immunohistochemistry, microarray, western blotting, and flow cytometry (FACS), but the present invention is not limited thereto.

Further, the present invention provides a method for inhibiting secretion of cancer-cell derived exosomes and expression of PD-L1 including administering IL-2, or an expression promoter or activator thereof to an individual.

### MODES FOR CARRYING OUT INVENTION

Hereinafter, the present invention will be described in more detail using examples. These examples are merely for illustrating the present invention in more specifically, and it would be apparent to those skilled in the art that the scope of the present invention is not limited by these examples.

### Example 1: Analysis of PD-L1 expression in melanoma cells using real-time polymerase chain reaction

After extracting mRNA (mRNA extraction kit, #9767; TaKaRa) from melanoma cells and melanoma cells pretreated with IL-2, the concentration of mRNA was measured using nanodrop (DS-11 Series Spectrophotometer; DeNovix). After synthesizing cDNA (SuperScript III First-Strand Synthesis System, Ref. 18080-051; invitrogen) with 100 ng of mRNA, the gene expression levels of mouse PD-L1 (primer set: Forward: AGT TCC CAAAAC ATG AGG AT (SEQ ID NO: 1), Reverse: CAC GTA CAA GTC CTT TGG AG (SEQ ID NO: 2)) and human PD-L1 (primer set: Forward: GGT CAT CCC AGAACT ACC TC (SEQ ID NO: 3), Reverse : ACG GAA GAT GAA TGT CAG TG (SEQ ID NO: 4)) were analyzed by performing real-time polymerase chain reaction.

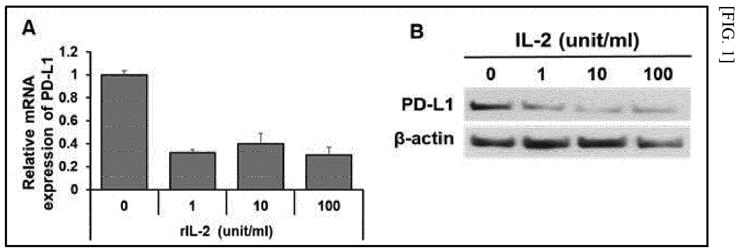

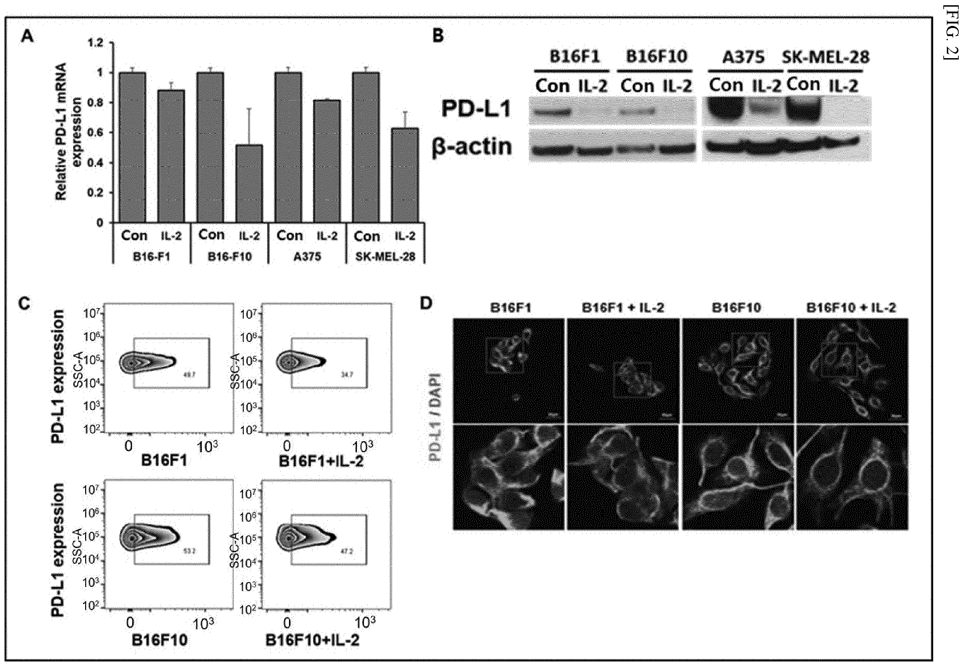

As a result, as shown in FIG. 1(A) and FIG. 2(A), it was determined that the gene expression of PD-L1 was reduced by about 50% in the melanoma cells pretreated with IL-2 compared to the control group.

### Example 2: Exosome purification

Control melanoma cells were seeded at a density of about 2 × 10⁶ cells/well, and cultured for 24 hours in DMEM medium without fetal bovine serum. After that, in order to isolate exosomes, each supernatant obtained from the cells was successively centrifuged at 300 × g, 2500 × g, and 10,000 × g. Then, the supernatant was filtered through a 0.2 µm syringe filter and centrifuged at 120,000 × g. The exosome pellet was resuspended in PBS and centrifuged again at 120,000 × g. The purified exosome pellet was resuspended in PBS or 1 × cell lysis buffer for the next experiment.

### Example 3: Characterization of purified exosomes

To check characteristics of purified exosomes using an electron microscope, the purified exosomes suspended in PBS were dropped on formvar carbon-coated nickel grids. After staining with 2% uranyl acetate, the grid was air-dried and the diameter structure was visualized using an HF-3300 (Hitachi) transmission electron microscope.

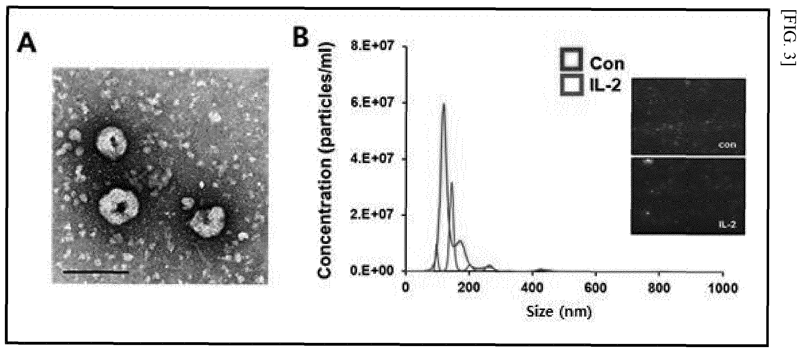

As a result, as shown in FIG. 3(A), the exosomes having a size of 50~150 nm were identified.

### Example 4: Analysis of exosomes derived from melanoma cells using nanoparticle tracking

After treating melanoma cells (B16F10: 5 × 10⁴ cells) with IL-2 at a concentration of 10 µg/ml and culturing for 48 hours, the size and concentration of exosomes derived from the culture supernatant were measured using fast video capture and a Nanosight LM10 (Malvern Instruments) equipped with particle tracking software.

As a result, as shown in FIG. 3(B), it was determined by nanoparticle tracking analysis that the number of exosomes decreased in the melanoma cells directly treated with IL-2 compared to the control group.

### Example 5: Analysis of exosomes derived from melanoma cells using Western blot

In order to re-verify the decrease in the number of exosomes derived from melanoma cells, the expression level of Rab protein involved in exosome secretion and expression was measured by Western blot. Cytoplasm or exosome proteins were separated by SDS-PAGE and transferred to a nitrocellulose membrane. Then, they were cultured with each of primary antibodies (ab18211; abcam/Rab5, ab50533; abcam/Rab7, ab55667; abcam/Rab 27a, #4970; Cell signalig/β-actin, ab10931; abcam/PD-L1), and with HRP conjugated secondary antibodies. Images were visualized using enhanced chemiluminescence (ECL) detection reagent (# 34095; Thermo Scientific, # RPN2209; GE Healthcare) and quantified using ECL hyperfilm (AGFA, Morstel).

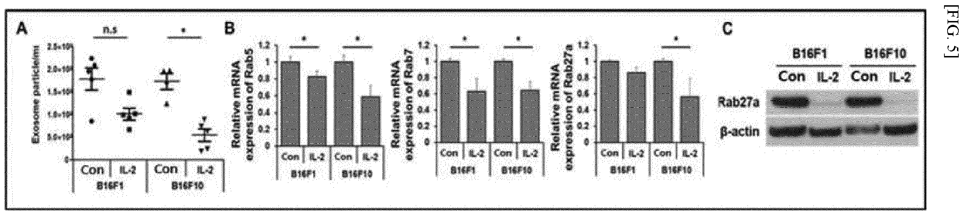

As a result, as shown in FIG. 5(C), it was determined that the protein expression of Rab 27a was significantly reduced in the melanoma cells treated with IL-2 compared to the control group.

In addition, to evaluate an effect of IL-2 in melanoma, the expression of PD-L1 (programmed death ligand 1) protein, which is known to be the most important for immune evasion of cancer cells, was measured.

As a result, as shown in FIG. 1 (B) and FIG. 2 (B), it was determined that the protein expression of PD-L1 was significantly reduced in the melanoma cells treated with IL-2 compared to the control group. This was re-verified through immunostaining as shown in FIG. 2(D).

Next, Western blot and flow cytometry were performed to evaluate whether the expression of exosomal PD-L1 derived from melanoma was regulated by IL-2.

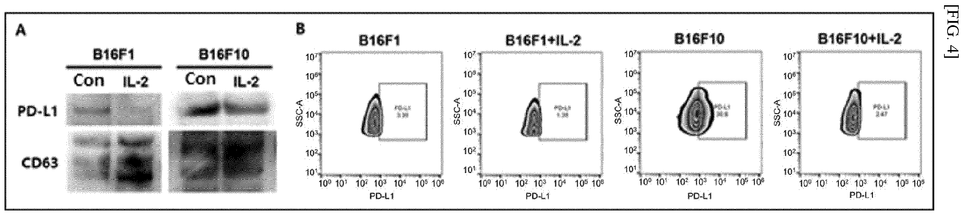

As a result, as shown in FIG. 4(A), it was determined by Western blot that the expression of exosomal PD-L1 in melanoma treated with IL-2 was significantly reduced compared to the control group, and as shown in FIG. 4(B), it was confirmed by revalidation using flow cytometry.

### Example 6: Co-culture assay

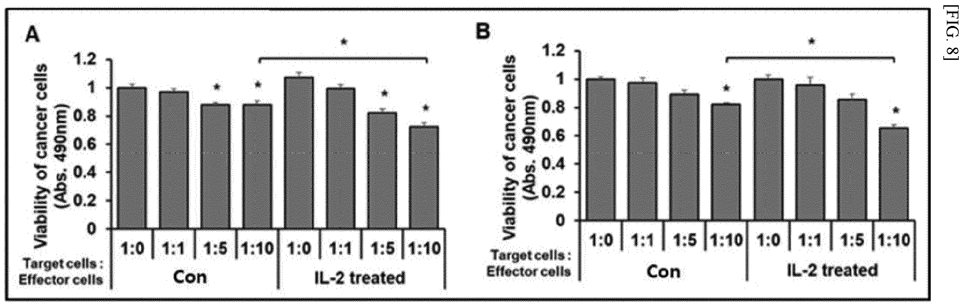

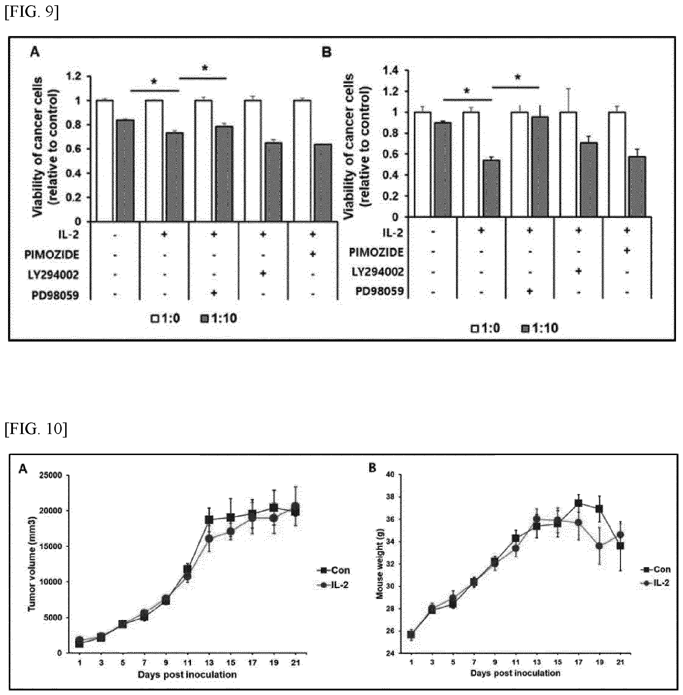

In order to prove the anticancer effect by increasing the activity of cytotoxic T cells along with the decrease in PD-L1 of melanoma cells by IL-2, as shown in FIG. 8(A) and FIG. 9(A), the viability of melanoma cells was checked after co-culture of IL-2 treated melanoma cells (B16F10) and cytotoxic T cells. Melanoma cells (B16F10-luc-g5) having a cytoluminescent gene were used and analyzed using IVIS spectrum (PerkinElmer).

Melanoma cells were seeded in a 96-well plate at a density of about 5 × 10³ cells/well, and melanoma cells pretreated with IL-2 were used as a control. After 48 hours of culture, the cells were cultured with different ratios of CTLL-2 cells (1:1 to 1:10). At the end of culture, MTS reagent was added to each well and cells cultured at 37°C for 2 hours. After removing MTS containing the supernatant from each well, absorbance was measured at 490 nm using a microplate reader.

As a result, as shown in FIG. 8(B) and FIG. 9(B), it was determined that the viability of melanoma cells was significantly reduced in the co-culture group of melanoma cells pretreated with IL-2 and cytotoxic T cells.

### Example 7: Analysis of decrease in PD-L1 expression of IL-2 in mouse model transplanted with melanoma

B16F10 mouse melanoma cells 1 × 10⁶ were subcutaneously injected into the right flank of balb/c mouse. After 10 days from the start of the experiment, PBS and rhIL-2 (50,000 IU/animal) were directly injected into the cancer tissue every day for 6 days. Thereafter, the cancer tissue (long axis ^{∗} short axis 2 ^{∗} 0.52) of the mouse was measured and compared once every 2 days using an engineering digital caliper.

As a result, as shown in FIG. 10, the cancer growth of the mouse injected with IL-2 was similar to that of the PBS control group. Thereafter, the same result as the weight analysis of the cancer tissue extracted from the mouse was shown (FIG. 10).

### Example 8: Analysis of regulation of PD-L1 expression in cancer of IL-2 in melanoma transplanted mouse model

After extracting the cancer tissue from the mouse of the above experiment and rapid cooling it using liquid nitrogen, RBC lysis buffer was used to remove red blood cells in the tissue, and then cells were obtained by centrifugation at 550 × g. The supernatant was then centrifuged continuously at 300 × g, 2,500 × g, and 10,000 × g. Then, the expression of PD-L1 was checked by Western blot and immunochemical staining using the isolated cells.

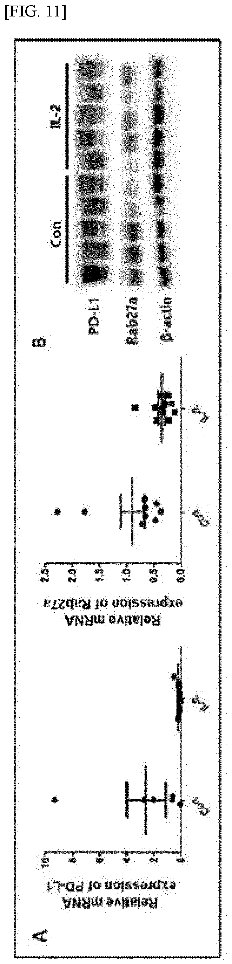

As a result, as shown in FIG. 11, it was determined that the expression level of PD-L1 in the mouse injected with IL-2 was significantly reduced compared to the control group.

The specific parts of the present invention have been described in detail above, and it is clear for those skilled in the art that these specific descriptions are merely preferred embodiments and the scope of the present invention is not limited thereto. Accordingly, the substantial scope of the present invention will be defined by the appended claims and their equivalents.

The scope of the present invention is indicated by the following claims, and all modifications or alternatives derived from the spirit and scope of the claims and their equivalents should be construed as being included in the scope of the present invention.

## Claims

1. A composition for enhancing an anticancer effect, comprising an inhibitor against expression of exosomal PD-L1 as an active ingredient.

2. The composition according to claim 1, wherein the inhibitor is IL-2, or an expression promoter or activator thereof.

3. The composition according to claim 2, wherein the IL-2 expression promoter or activator is any one selected from the group consisting of a compound, peptide, aptamer, and antibody that specifically binds to IL-2 protein.

4. The composition according to claim 2, wherein the IL-2, or the expression promoter or activator thereof suppresses immune evasion and enhances the anticancer effect by reducing secretion of cancer cell-derived exosomes and expression of PD-L1.

5. The composition according to claim 1, wherein the cancer is selected from the group consisting of melanoma, skin cancer, lung cancer, liver cancer, stomach cancer, pancreatic cancer, bone cancer, head or neck cancer, uterine cancer, ovarian cancer, breast cancer, fallopian tube carcinoma, endometrial carcinoma, cervical carcinoma, vaginal carcinoma, vulvar carcinoma, Hawkins' disease, esophageal cancer, small intestine cancer, colorectal cancer, colon cancer, rectal cancer, perianal cancer, endocrine adenocarcinoma, thyroid cancer, parathyroid cancer, adrenal cancer, soft tissue sarcoma, urethral cancer, penile cancer, prostate cancer, chronic or acute leukemia, lymphocyte lymphoma, bladder cancer, kidney or ureter cancer, renal cell carcinoma, renal pelvic carcinoma, central nervous system tumor, primary central nervous system lymphoma, spinal cord tumor, brainstem glioma, and pituitary adenoma.

6. The composition according to claim 1, wherein the composition is administered in combination with one or more anticancer agents.

7. A pharmaceutical composition comprising the composition for enhancing an anticancer effect of any one of claims 1 to 6 as an active ingredient.

8. A method for screening an inhibitor against secretion of cancer cell-derived exosomes and expression of PD-L1, comprising:
treating samples isolated from an individual suspected of having a cancer disease with candidate drugs;
measuring expression or activity level of IL-2 in the samples treated with the candidate drugs; and
selecting one of the candidate drugs that increases the expression or activity level of IL-2 as compared to a control sample.

9. The method according to claim 8, wherein the expression or activity level of IL-2 is measured by one or more selected from the group consisting of reverse transcription-polymerase chain reaction (RT-PCR), enzyme-linked immunosorbent assay (ELISA), radioimmunoassay, immunohistochemistry, microarray, western blotting, and flow cytometry (FACS).

10. A method of inhibiting secretion of cancer cell-derived exosomes and expression of PD-L1, comprising administering IL-2, or an expression promoter or activator thereof to an individual.
